Europäisches Patentamt

European Patent Office

Office européen des brevets

(11) Veröffentlichungsnummer : **0 068 377**
B1

# (12) EUROPÄISCHE PATENTSCHRIFT

(45) Veröffentlichungstag der Patentschrift :
28.08.85

(21) Anmeldenummer : 82105410.3

(22) Anmeldetag : 21.06.82

(51) Int. Cl.⁴ : **B 01 D 53/36, C 07 C 47/04**

(54) **Verfahren zur katalytischen Oxydation von Abgasen aus Formaldehydanlagen.**

(30) Priorität : 01.07.81 AT 2918/81

(43) Veröffentlichungstag der Anmeldung :
05.01.83 Patentblatt 83/01

(45) Bekanntmachung des Hinweises auf die Patenterteilung : 28.08.85 Patentblatt 85/35

(84) Benannte Vertragsstaaten :
AT BE CH DE FR GB IT LI NL SE

(56) Entgegenhaltungen :
FR-A- 2 063 216
FR-A- 2 471 805

(73) Patentinhaber : Kanzler, Walter, Dipl.Ing.
Stiftingtalstrasse 165d
A-8010 Graz (AT)

Schedler, Johannes, Dipl.Ing.
Stiftingtalstrasse 165d
A-8010 Graz (AT)

(72) Erfinder : Kanzler, Walter, Dipl.Ing.
Stiftingtalstrasse 165d
A-8010 Graz (AT)
Erfinder : Schedler, Johannes, Dipl.Ing.
Stiftingtalstrasse 165d
A-8010 Graz (AT)

## Beschreibung

Die Erfindung betrifft ein Verfahren zur Reinigung von Abgasen aus Formaldehydanlagen, die im wesentlichen aus Sauerstoff, Stickstoff, Kohlenmonoxyd, Kohlendioxyd, Wasserstoff, Methanol, Dimethyläther und Formaldehyd bestehen. Bei den Verfahren zur Oxydation von Methanol zu Formaldehyd enthält das Abgas nach dem Formaldehyd-Absorber meist noch größere Mengen von Veruneinigungen, insbesondere Kohlenmonoxyd und Formaldehyd.

Während die Abgase aus Anlagen, die nach dem Silberkontaktverfahren arbeiten, durch den hohen Gehalt an Wasserstoff (bis zu 20 Vol.% Wasserstoff, Heizwert ca. 2 000 KJ/m$^3$) in einer Fackel verbrannt werden können oder zur Erzeugung von Dampf Verwendung finden, sind die bisher vorgeschlagenen Lösungen für das Abgasproblem nach dem Formox-Verfahren mit metalloxydischen Kontakten noch nicht wirtschaftlich gelöst.

Diese Abgase enthalten bis zu 3 000 ppm Formaldehyd und bis zu 2 Vol.% Kohlenmonoxyd, während der behördlich festgesetzte Wert von maximal 20 ppm Formaldehyd im Abgas meist nur durch aufwendige Verfahren erreicht wird. So können diese Abgase durch Verbesserungen der Absorptionstürme auf größere Reinheit gebracht werden oder aber durch nachgeschaltete chemische Verfahren. Die Verbesserung der Absorptionstürme ist meist durch zu hohe Temperatur der Waschflüssigkeiten begrenzt und die Vergrößerung der Kolonnen bringt zusätzlichen Druckverlust.

Ferner werden die Abgase nach dem Formox-Verfahren auch unter Zusatz von Erdgas verbrannt, was hohe Brennstoffkosten verursacht.

Es werden auch Naßabsorptionsverfahren zur Entfernung von Formaldehyd vorgeschlagen und gebaut, wobei der Waschflüssigkeit anorganische Salze oder Ammoniak zugegeben werden. Alle diese Verfahren sind teuer und verursachen zusätzliche Betriebskosten.

Der Erfindung liegt daher die Aufgabe zugrunde, ein kostengünstiges Verfahren zur Reinigung dieser Abgase zu finden und die Konzentration von Formaldehyd im Abgas möglichst weit unter 20 ppm zu senken.

Eine weitere Aufgabe der Erfindung war es, den Kohlenmonoxydgehalt im Abgas ebenfalls zu reduzieren.

Die Aufgabe wird erfindungsgemäß dadurch gelöst, daß man das Abgas in einem Vorwärmer auf 140°-200 °C aufheizt, das vorgewärmte Abgas über ein Katalysatorbett leitet, das aus einem inerter, anorganischen, hochporösen Trägermaterial besteht, auf essen äußere Oberfläche eine aus 0,1 bis 0,5 Gewichts-% Platin bestehende Platinschicht aufgebracht ist, worauf das am Katalysator durch die freiwerdende Reaktionswärme auf 250°-450 °C aufgewärmte Abgas in einem Wärmetauscher unter Energiegewinnung in Form von Wasserdampf oder Thermoöl auf 150°-300 °C abgekühlt wird und die restliche Wärme des Abgases entweder direkt dem Vorwärmer oder einem Kühlmedium in einem Wärmetauscher zugeführt wird, der das eintretende Abgas in Vorwärmer aufwärmt. Es ist hinreichend bekannt, organische Verbindungen in Abgasen katalytisch zu oxydieren. Diese Verfahren werden meist bei Temperaturen über 400 °C durchgeführt und sind sehr energieintensiv.

Es ist daher überraschend, daß Formaldehyd schon bei den erfindungsgemäßen Temperaturen an Platinkatalysatoren zu Kohlendioxyd und Wasser oxydiert werden kann und zwar mit großer Geschwindigkeit und großem Umsatz. Dabei hat es sich gezeigt, daß es nicht erforderlich ist, reines Platin zu verwenden, sondern daß es wesentlich ist, für den Katalysator hochporöse Oberflächen zu schaffen, die mit 0,1 bis 0,5 Gewichts % Platin besetzt sind. Als Trägermaterial kann Aluminiumoxyd oder Bariumsulfat in Form von 2-10 mm großem Granulat oder anderen Fastkörpern eingesetzt werden. Größere Platin-Gehalte verbessern die Reaktion im Verhältnis zum Preis nur unwesentlich.

Gemäß der Erfindung zeigte es sich auch, daß es möglich ist, Kohlenmonoxyd schon bei Temperaturen ab 140 °C zu Kohlendioxyd zu oxydieren.

Da die Konzentration von Kohlenmonoxyd im Abgas aber meist zwischen 0,1 und 2 Vol.% liegt, ist es von wesentlicher Bedeutung, die Reaktionswärme der Reaktion von Kohlenmonoxyd zu Kohlendioxyd zu nützen und sowohl das Katalysatorbett, als auch das Abgas aus dem Absorber damit aufzuheizen, wodurch die Reaktionsgeschwindigkeit wesentlich vergrößert werden kann.

Die Aufheizung des Abgases erfolgt vorzugsweise durch einen eigenen Kreislauf, wobei mit einem Kühlmedium das heiße Abgas mit einem Wärmetauscher gekühlt wird und das warme Kühlmedium das kalte Abgas aufwärmt. Auf diese Weise können Reaktionstemperaturen bis 450 °C erreicht werden.

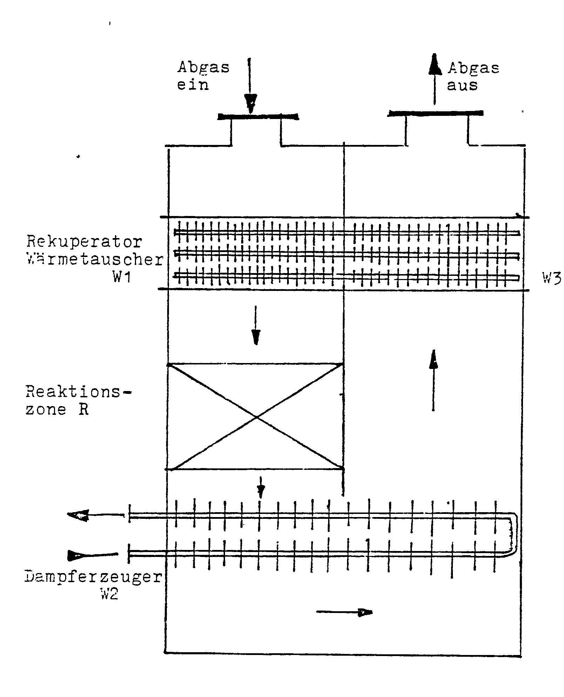

Da bei den meisten Formaldehyd-Anlagen nach dem Formox-Verfahren die Konzentration der organischen Verunreinigungen im Abgas (Formaldehyd, Methanol, Kohlenmonoxyd und Dimethyläther u. a.) zwischen 1 und 2 Vol.% liegt, kann nach der Reaktionszone R (s. Abbildung 1) der Gasstrom zunächst auf ca. 200 °C abgekühlt werden, wobei die Wärme z. B. zur Dampferzeugung Verwendung findet, während die verbleibende Wärme, die bei der Abkühlung des Gases frei wird, zur Vorheizung des in die Reaktionszone eintretenden Abgases auf ca. 150 °C Verwendung findet.

Als Kühlmedium kann dabei Thermoöl verwendet werden oder bei größeren Wärmeüberschüssen auch nach dem Katalysatorbett Dampf erzeugt werden.

Beispiel

40 000 M³/h Abgas mit 36 °C aus einem Formaldehyd-Absorber nach dem Formox-Verfahren enthalten :

| | |
|---|---|
| $N_2$ | 85,23 Vol.% |
| $O_2$ | 11 Vol.% |
| Methanol | 0,3 Vol.% |
| Formaldehyd | 0,1 Vol.% |
| Dimethyläther | 0,1 Vol.% |
| $CO_2$ | 0,17 Vol.% |
| CO | 0,8 Vol.% |
| $H_2O$ | 2,3 Vol.% |

Das aus der Absorptionskolonne austrentende Abgas wird durch den Vorwärmer W1 geleitet, in dem das Abgas im Gegenstrom z. B. mit einem Thermoöl als Wärmeträger auf 150 °C erwärmt wird.

Der Vorwärmer W1 kann erfindungsgemäß ein Wärmetauscher mit Wärmerohren sein.

In der Reaktionszone $R_1$ die aus einer 0,1 m hohen Schicht mit porösem Granulat mit einem Durchmesser von 8 mm besteht (0,2 Gew.% Platin auf Aluminiumoxyd oder Bariumsulfat), erhitzt sich das Abgas durch die Reaktionswärme auf 450 °C.

Dabei reduziert sich die Konzentration von Formaldehyd auf 10 ppm und die von Kohlenmonoxyd auf 0,004 Vol.%. Im Wärmetauscher W2 wird das Abgas auf ca. 210 °C abgekühlt, wobei die Wärme in Form von 20 bar Sattdampf gewonnen wird und aus der Anlage abgegeben werden kann. Im Wärmetauscher W3 wird die zur Vorwärmung des Abgases auf 150 °C benötigte Wärmemenge bei der Abkühlung des Abgases von 210 °C auf 95 °C im Gegenstrom rückgewonnen, so daß zur Vorwärmung in W1 das Thermoöl mit einer Temperatur von ca. 180 °C zur Verfügung steht.

**Patentansprüche**

1. Verfahren zur Reinigung von Abgasen aus Formaldehydanlagen, die im wesentlichen aus Sauerstoff, Stickstoff, Kohlenmonoxyd, Kohlendioxyd, Wasserstoff, Methanol, Dimethyläther und Formaldehyd bestehen, dadurch gekennzeichnet, daß man das Abgas in einem Vorwärmer W1 auf 140°-200 °C aufheizt, das vorgewärmte Abgas über ein Katalysatorbett leitet, das aus einem inerten, anorganischen, hochporösen Trägermaterial besteht, auf essen äußere oberfläche eine aus 0,1 bis 0,5 Gewichts-% Platin bestehende Platininschicht aufgebracht ist, worauf das am Katalysator durch die freiwerdende Reaktionswärme auf 250°-450 °C aufgewärmte Abgas in einem Wärmetauscher W2 unter Energiegewinnung in Form von Wasserdampf oder Thermoöl auf 150°-300 °C abgekühlt wird und die restliche Wärme des Abgases entweder direkt dem Vorwärmer W1 oder einem Kühlmedium in einem Wärmetauscher W3 zugeführt wird, welches das eintretende Abgas im Vorwärmer W1 aufwärmt.

2. Verfahren nach Anspruch 1, dadurch gekennzeichnet, daß als Trägermaterial Aluminiumoxyd oder Bariumsulfat in Form von 2-10 mm großem Granulat oder anderen Festkörpern eingesetzt wird.

3. Verfahren nach Anspruch 1 bis 2, dadurch gekennzeichnet, daß als Vorwärmer ein Wärmetauscher mit Wärmerohren eingesetzt wird.

**Claims**

1. A process for the purification from flue gases of formaldehyde plants, essential consisting of oxygen, nitrogen carbon monoxide, carbon dioxide, hydrogen, methanol, dimethylether, thereby characterized, that the flue gas is heated in a preheater W1 to 140-200 °C, the preheated flue gas is passed through a catalyst layer, consisting of an inert, inorganic carrier with high porosity on which surface a layer is brought up consisting of 0.1-0.5 wt % platinum. The gas stream heated by the heat of reaction to 250-450 °C is cooled in a heat exchanger W2 to 150-300 °C whereby the released heat is recovered as steam or with thermo oil and the remaining heat of the flue gas is passed either directly to the preheater W1 or to a cooling agent in a heat exchanger W3, which is heating the feed gas in preheater W1.

2. A process according claim 1, thereby characterized, that $Al_2O_3$ or $BaSO_4$ is used as carrier in form of granulat or other solids with 2-10 mm size.

3. A process according claim 1 and 2, thereby characterized, that a heat exchanger with heat pipes is used as preheater.

**Revendications**

1. Procédé pour la purification des polluants dans les appareils de formaldéhyde consistant essentiellement d'oxygène, d'azote, de monoxyde de carbone, de dioxyde de carbone, d'hydrogène, de méthanol, de diméthyléther et de formaldéhyde est caractérisé par le fait qu'on chauffe le polluant dans un préchauffage W1 à une température de 140° à 200 °C, qu'on fait passer le polluant préchauffé à travers un lit catalytique consistant d'un matériel-porteur inerte, anorganique et extrémement poreux sur la surface extérieure duquel on a mis une couche de 0,1 à 0,5 poids % de platine. Puis on réfirgère le polluant chauffé à une température de 250° à 450 °C sur le catalyseur par la chaleur réactrice libérée dans un échangeur de chaleur W2 par production énergétique sous forme de vapeur d'eau ou de thermo-huile à une température de 150° à 300 °C. On fait passer la chaleur restante du polluant ou directement au préchauffage W1 ou à un médium réfrigérant dans un échangeur de chaleur W3. Ce médium réfrigérant réchauffe le polluant entrant dans le réchauffage W1.

2. Le procédé selon la revendication 1 est caractérisé par le fait que le matériel-porteur consiste en oxyde d'aluminium ou de sulfate de barium sous forme de granules à une grandeur de 2-10 mm ou d'autre structure rigide.

3. Le procédé selon la revendication 1 et 2 est caractérisé par le fait que l'on utilise un échangeur de chaleur avec des tuyaux de chaleur comme préchauffage.

Abgas
ein

Abgas
aus

Rekuperator
Wärmetauscher
W1

W3

Reaktionszone R

Dampferzeuger
W2

ABBILDUNG 1